# EUROPEAN PATENT APPLICATION

(11) **EP 4 574 969 A1**
(43) Date of publication of application: **25.06.2025**
(21) Application number: 23219869.7
(22) Date of filing: 22.12.2023
(51) Int. Cl.: C12N 5/0786, C12M 1/00, C12M 1/26

(54) **A METHOD OF PRODUCING DIFFERENTIATED CELLS FROM PLURIPOTENT STEM CELLS, CELL POPULATIONS OBTAINED BY THE METHOD, CORRESPONDING PHARMACEUTICAL COMPOSITIONS AND A SYSTEM FOR PRODUCING DIFFERENTIATED CELLS FROM PLURIPOTENT STEM CELLS**

(71) Applicant: Sartorius Stedim Biotech GmbH, 37079 Göttingen (DE)
(72) Inventor: RADDATZ, Lukas, 37079 Göttingen (DE); AUSTERJOST, Jonas, 37079 Göttingen (DE)
(74) Representative: Schiweck Weinzierl Koch Patentanwälte Partnerschaft mbB

(57) **Abstract**

The present invention inter alia relates to a method of producing differentiated cells from pluripotent stem cells, comprising providing at least one multicellular 3D aggregate derived from pluripotent stem cells cultured in a first suspension culture, inducing differentiation of the cells forming the at least one multicellular 3D aggregate, allowing a continuous release of differentiated cells from the multicellular 3D aggregate into the first suspension culture; and continuously separating the released differentiated cells from the first suspension culture. The present invention also relates to a cell obtained by the method and to a pharmaceutical composition comprising the cells. The invention further relates to a corresponding system for producing differentiated cells from pluripotent stem cells.

## Description

### FIELD OF THE INVENTION

The present invention relates to a method of producing differentiated cells from pluripotent stem cells, comprising the steps of providing at least one multicellular three-dimensional (3D) aggregate derived from pluripotent stem cells cultured in a first suspension culture; inducing differentiation of the cells forming the at least one multicellular 3D aggregate; and allowing a continuous release of differentiated cells from the multicellular 3D aggregate into the first suspension culture. The invention further relates to cell populations obtained by the method, to a pharmaceutical composition comprising the cells, and to a system for producing differentiated cells from pluripotent stem cells, comprising at least one bioreactor chamber, wherein the at least one bioreactor chamber comprises at least one fluid inlet and at least one fluid outlet; further wherein the system comprises a separation unit in fluid communication with the at least one bioreactor chamber, wherein the separation unit comprising at least one fluid inlet and one or more fluid outlets; and wherein a first fluid outlet of bioreactor chamber being in fluid communication with a first inlet of the separation unit.

### BACKGROUND OF THE INVENTION

The discovery of induced pluripotent stem cell (iPSC) technology has opened up unprecedented opportunities in e.g. treatment of diseases, regenerative medicine, disease modelling and drug discovery. As iPSC reprogramming technology is still relatively new, challenges remain - especially with respect to cell proliferation and differentiation. For example, partially or terminally differentiated cells are typically manufactured by differentiating induced pluripotent stem cells (iPSCs) in complex multistep processes. Such traditional methods lack precision and may result in suboptimal yields, affecting the overall efficacy and safety of advanced therapy products.

Recently, it was discovered that cell clusters/aggregates with partially differentiated iPSCs referred to as embryoid bodies in suspension continuously release differentiated cells such as macrophages by providing a cytokine cocktail for their differentiation which can be harvested once a week. While such differentiated cells can be harvested regularly, e.g. on weekly basis, such process is conducted manually and at low scale. Hence, there remains a need for improved methods and systems to produce differentiated cells from pluripotent cells. Specifically, there is a need to increase efficiency and safety.

### SUMMARY OF THE INVENTION

This object is inter alia accomplished by the methods, cell populations, pharmaceutical composition, uses and systems having the features of the respective independent claims.

In a first aspect, the invention provides a method of producing differentiated cells from pluripotent stem cells, comprising
- providing at least one multicellular three-dimensional (3D) aggregate derived from pluripotent stem cells cultured in a first suspension culture;
- inducing differentiation of the cells forming the at least one multicellular 3D aggregate;
- allowing a continuous release of differentiated cells from the at least one multicellular 3D aggregate into the first suspension culture; and
- continuously separating the released differentiated cells from the first suspension culture.

By continuously separating the released differentiated cells from the first suspension culture (comprising the at least one multicellular 3D aggregate), potential side effects during the prolonged co-culture of the released differentiated cells in the first suspension culture with the cell clusters/aggregates with partially differentiated iPSCs according to prior art methods is prevented/reduced. Furthermore, continuously separating the released differentiated cells from the first suspension culture (comprising the at least one multicellular 3D aggregate) enables obtaining of differentiated cells having a more homogeneous level of differentiation compared with cells obtained in a non-continuous, batch-wise manner. For example, it has been shown that the presence of neighboring cells in multicellular aggregates has a significant impact on the proliferative and differentiated state of cells. [Liu W.F. and Chen C.S.; 2007]. Since the earliest events during differentiation have been described to be epigenetic changes which may occur continuously and short-termed during early differentiation [Schmidt M et al.; 2022], the continuous separation of differentiated cells from the cell clusters/aggregates with partially differentiated iPSCs may enable the obtaining of cells having a more homogeneous differentiation state, thereby reducing the risk of potential side effects.

In a second aspect, the invention provides a cell population obtained by any one of the methods according to the invention.

As differentiated cells of the obtained cell population have been separated from the first suspension culture (comprising the at least one multicellular 3D aggregate) in the continuous separation process according to the invention, without being bound to theory, it is envisaged that e.g. the epigenetic state of the cells in the obtained cell population ressembles a more homogenous, early differentiation state in comparison to cell populations obtained with prior art methods disclosing batch-wise obtaining of differentiated cells.

In a third aspect, the invention provides a pharmaceutical composition comprising the cells according to the invention.

In a fourth aspect, the invention provides the cell population obtained by the method according to the invention for use in treating or preventing a disease.

In a fifth aspect, the invention provides a separation unit for separating differentiated cells from pluripotent cells, wherein the separation unit comprises a porous-structured and/or micro-holed unit, wherein the porous-structured and/or micro-holed unit is construed to being capable of separating a differentiated cell from a multicellular 3D aggregate.

In a sixth aspect, the invention provides a system for producing differentiated cells from pluripotent stem cells, comprising
- a bioreactor comprising at least one bioreactor chamber; the at least one bioreactor chamber having at least one fluid inlet and at least one fluid outlet;
- a separation unit in fluid communication with the at least one bioreactor chamber; the separation unit comprising at least one fluid inlet and one or more fluid outlets;

wherein a first fluid outlet of bioreactor chamber is continuously in fluid communication with a first inlet of the separation unit;
further wherein the separation unit comprises a porous-structured and/or micro-holed unit, wherein the porous-structured and/or micro-holed unit is construed to being capable of separating a differentiated cell from a multicellular 3D aggregate;
or wherein the separation unit comprises a sedimentation unit, wherein the sedimentation unit is construed to being capable of separating a differentiated cell from a at multicellular 3D aggregate.

All aspects of the invention provide the above-described advantages and improvments related to the prevention /reduction of potential side effects that may occur during a prolonged co-culture of the released differentiated cells in the first suspension culture with the cell clusters/aggregates with partially differentiated iPSCs according to prior art methods.

### BRIEF DESCRIPTION OF THE DRAWINGS

The invention will be better understood with reference to the detailed description when considered in conjunction with the non-limiting examples and the drawings, in which:
**Fig. 1** shows a schematic representation of a system for producing differentiated cells from pluripotent stem cells in accordance with one embodiment of the present invention.
**Fig. 2** shows a schematic representation of a system for producing differentiated cells from pluripotent stem cells in accordance with another embodiment of the present invention.
**Fig. 3** shows a schematic representation of a system for producing differentiated cells from pluripotent stem cells in accordance with a further embodiment of the present invention.
**Fig. 4** shows a scanning electron microscope image of an exemplary micro-holed unit of the system in accordance with some embodiments of the present invention; wherein **Fig. 4A** shows a micro-holed unit made of ceramic material with hole size of about 200 µm; and wherein **Fig. 4B** shows a micro-holed unit made of epoxy-resin material with hole size about 10 µm.

### DETAILED DESCRIPTION OF THE INVENTION

As explained above, in a first aspect the invention is directed to a method of producing differentiated cells from pluripotent stem cells, comprising
- providing at least one multicellular 3D aggregate derived from pluripotent stem cells cultured in a first suspension culture;
- inducing differentiation of the cells forming the at least one multicellular 3D aggregate;
- allowing a continuous release of differentiated cells from the at least one multicellular 3D aggregate into the first suspension culture; and
- continuously separating the released differentiated cells from the first suspension culture.

The term "suspension culture" as used herein is a type of cell culture in which single cells, small aggregates of cells, or cell aggregates/clusters, e.g. embryoid bodies, are allowed to function and multiply in a preferably agitated growth medium, thus forming a suspension (c.f. the definition in chemistry: "small solid particles suspended in a liquid"). A suspension culture differs from adherent culture, in which the cells are attached to a cell culture container, which may be coated with proteins of the extracellular matrix (ECM). In suspension culture, preferably no proteins of the ECM are added to the cells and/or the culture medium. The suspension culture preferably is essentially free of solid particles such as beads, microspheres, microcarrier particles and the like; cells or cell aggregates are no solid particles within this context.

In literature, multicellular 3D aggregates derived from pluripotent stem cells (PSC) have been described as "Embryoid bodies" (EBs). Specifically, while, originally, EBs have been described that were derived from embryonic stem cells, recently, also multicellular 3D aggregates derived from e.g. induced pluripotent stem cells (iPSC) have been referred to as EBs. Thus, a mutlicellular 3D aggregate according to the present invention may be refered to as EB and/or may comprise (at least one) EBs. A multicellular 3D aggregate according to the present invention may comprise cell types of all three germ layers and may typically be rounded. PSCs as used herein refers to cells that are able to differentiate into every cell type of the body. As such, PSCs offer the unique opportunity to be differentiated into essentially any tissue or organ. Currently, the most utilized pluripotent cells are embryonic stem cells (ESC) or induced pluripotent stem cells (iPSC). Thus, pluripotent cell types from which mutlicellular 3D aggregate according to the present invention may be derived include embryonic stem cells (ESCs) derived from the blastocyst stage of embryos, e.g., from mouse (mESC), primate, and human (hESC) sources. Additionally, mutlicellular 3D aggregate according to the present invention (e.g. EBs) may be formed from PSC derived through other techniques, including e.g. somatic cell nuclear transfer or e.g. the reprogramming of somatic cells to yield iPSCs. Resulting induced pluripotent cells (iPSC) show a very similar behavior as ESC and, importantly, are also able to differentiate into every cell of the body. Methods for generating EBs are well known in the art. Thus, for example, the mutlicellular 3D aggregates of the invention may be obtained by a method comprising cultivating pluripotent stem cells such as iPSC in a suspension culture for a sufficient period of time to produce mutlicellular 3D aggregates (e.g. embryoid bodies). In an embodiment, the method according to the present invention may comprise the step of producing the at least one mutlicellular 3D aggregate.

In the present invention, "differentiated cells" refer to any cells that have been differentiated and induced to differentiate from pluripotent stem cells to specific types of cells. Differentiated cells may include adherent cells and non-adherent cells, such as blood cells, that make up tissues such as cardiomyocytes and skeletal myoblasts. Non-limiting examples of differentiated cells include cardiomyocytes, muscle-based cells such as skeletal myoblasts, neuronal cells such as neuronal cells, oligodendrocytes, dopamine-producing cells, retinal cells such as retinal pigment epithelial cells, hematopoietic cells such as blood cells, bone marrow cells, T cells, NK cells, NKT cells, dendritic cells, other immune related cells such as B cells, hepatocytes, pancreatic beta cells, cells that make up organs such as kidney cells, chondrocytes, germ cells, etc. progenitor cells and somatic stem cells that differentiate into these cells, and the like. Typical examples of such progenitor cells and somatic stem cells include, for example, mesenchymal stem cells in cardiomyocytes, multipotent cardiac progenitor cells, unipotent cardiac progenitor cells, neural stem cells in neural cells, hematopoietic stem cells in hematopoietic and immune-related cells, lymphoid stem cells, and the like. The differentiation induction of pluripotent stem cells can be carried out using any known technique. For example, differentiation induction from pluripotent stem cells to cardiomyocytes can be performed based on the techniques described in the art, e.g. by [Mummery C. L. et al., 2012], and differentiation induction from pluripotent stem cells to hematopoietic, in particular, myeloid cells, has also been described in the art.

As described above, recently, it has been demonstrated that cell clusters/aggregates with partially differentiated iPSCs in suspension continuously release differentiated cells into the cell culture suspension. In accordance with the present invention, the method comprises a step of allowing such continuous release of differentiated cells from the at least one multicellular 3D aggregate into the first suspension culture.

The first suspension culture may be continuously agitated, for example stirred. Agitating (e.g. stirring) may be controlled to reduce any potential effect of mechanical cues on the differentiation of pluripotent stem cells.

According to an embodiment, the released differentiated cells may comprise single differentiated cells, or cell aggregates of the differentiated cells.

According to an embodiment, the at least one multicellular 3D aggregate may comprise the pluripotent stem cells, or progenitor cells derived from the pluripotent stem cells, or a mixture thereof.

For example, the stem cells may be primate stem cells such as human stem cells.

According to an embodiment, the produced differentiated cells may be partially or terminally differentiated cells.

According to an embodiment, continuously separating the differentiated cells from the first suspension culture comprises the step of separating the differentiated cells from the at least one multicellular 3D aggregate. For example, the expression "first suspension culture" relates to the suspension culture.

In accordance with an embodiment, the step of separating the differentiated cells from the at least one multicellular 3D aggregate may comprise passing the first suspension culture through a separation unit, wherein the separation unit may be construed to separate components of the first suspension culture according to size. The inclusion of the separation unit simplifies/enables carrying out the entire method in a closed system, thereby improving safety of the process. Furthermore, the efficiency is increased through automation of the separation step inside the system.

Alternatively, in accordance with an embodiment, the step of separating the differentiated cells from the at least one multicellular 3D aggregate may comprise separating components of the first suspension culture according to density.

In embodiments of the present application, in which the step of separating the differentiated cells from the at least one multicellular 3D aggregate may comprise separating components of the first suspension culture according to density, the separation unit may comprise a sedimentation unit.

For example, a fraction of the first suspension culture may be continuously transferred into a container of the sedimentation unit, wherein the container may comprise at least one opening at the bottom. Inside the container, multicellular 3D aggregates, single differentiated cells and/or differentiated cells in form of small aggregates may be allowed to settle down by gravitation. Multicellular 3D aggregates with higher density than single differentiated cells and smaller aggregates of cells may be maintained in the first suspension culture e.g. by transferring multicellular 3D aggregates through the opening at the bottom. Single differentiated cells and/or smaller aggregates of differentiated cells may continuously be separated from the first suspension culture e.g. by transferring the single differentiated cells and/or small aggregates of differentiated cells after separation by size from the container into a collection container, e.g. by transferring single differentiated cells and/or small aggregates of differentiated cells through an opening within a top fraction of the container which may not accessible for multicellular 3D aggregates due to their higher density in the separation process.

In an embodiment of the present invention, in which the separation unit may be construed to separate components of the first suspension culture according to size, the separation unit may comprise a porous-structured and/or micro-holed unit, such as a filter or membrane.

According to an embodiment, the porous-structured and/or micro-holed unit may be monolithic. The expression "monolithic" as used herein is understood to refer to a feature or feature(s) consisting of a single piece of a particular material. It can specifically refer to a homogeneous material.

According to an embodiment, the porous-structured and/or micro-holed unit may comprise a hole diameter size of less than about 1000 µm, or less than about 500 µm, or less than about 200 µm, or less than about 150 µm, or less than about 100 µm; and/or wherein the porous-structured and/or micro-holed unit may comprise a hole diameter size of between about 100 µm and 800 µm, between about 150 and 800 µm, between about 100 µm and 500 µm, between about 300 µm and 500 µm, between about 100 µm and 300 µm, between about 100 to 250 µm, between about 100 and 200 µm, or between about 100 and 150 µm.

For example, these hole diameter sizes allow for efficient and uniform separation of e.g. single sifferentiated cells from the at least one multicellular 3D aggregate. Undesireable shear effects that may occur if multicellular 3D aggregates interact with too small hole sizes of micro-holed units are avoided.

According to an embodiment, the porous-structured and/or micro-holed unit may be produced:
- by additive manufacturing, such as Vat Photopolimerization (VP) or Powder Bed Fusion (PBF), e.g. Lithography-based Ceramics Manufacturing (LCM), Selective Laser Sintering (SLS), Stereolithography (SLS), or Digital Light Synthesis (DLS),
- by sintering, or
- by laser cutting.

According to some embodiments, the porous-structured and/or micro-holed unit may be made of a ceramic material, a stainless-steel material, a metal alloy material, an epoxy resin material, or a polymer material such as a thermoplastic material.

Advantageously, the use of additive manufacturing as herein provided allows e.g. the provision of better defined cut-off values and/or smoother micro-hole edges, which are important parameters production of micro-holed units regarding suitability for retaining multicellular 3D aggregates in the first suspension culture without damaging their structure and/or function. For example, undesireable shear effects may occur if multicellular 3D aggregates interact with too small hole sizes of micro-holed units. Such shear effects are avoided when using the defined hole sizes of micro-holed units as described herein. Furthermore, this technology allows to change hole sizes according to the expected size of multicellular 3D aggregates which have to be retained, allows to select a specific orientation of holes in the micro-holed unit, and may introduce a porosity into the micro-hole unit. Without being bound to theory, it is believed that usage of a micro-holed unit produced by additive manufacturing methods as herein provided may reduce the risk of any undesired activation of the multicellular 3D aggregates in the first suspension culture. For example, it has been described earlier that mechanical forces interfere with the development of embryonic tissues [Campas O., 2016]. It is thus important to control mechanical stimuli, including compression, tensile, or shear forces during culturing of the multicellular 3D aggregates in order to address the effect of mechanical cues on the differentiation of pluripotent stem cells.

Thus, for example, a standard deviation of the hole sizes in the micro-holed unit may be less than 20 µm, less than 15 µm, or less than 10 µm, or a standard deviation of the hole sizes may be between 2 and 15 µm, or between 5 and 15 µm, or between 5 and 10 µm, or between 10 and 15 µm, or between 10 and 12 µm. As explained above, using hole-sized having such controlled low standard variations reduces the risk of inconsistent conditions of the multicellular 3D aggregates during the process, thus increasing consistency and safety of the process.

The term "continuously separating" as used herein in scope of separating culture means separating the released differentiated cells from the first suspension continuously or periodically/intermittently over a period of time. Typically, the differentiated cells are separated as a suspension including a surrounding liquid, e.g. cell culture media. The volume of cells and preferably a fraction of the surrounding liquid is replaced by adding substantially the same volume of replacement liquid culture medium to the first suspension culture. Hence, such process may be performed as perfusion culture which is a term of art. When continuously separating means separating the released differentiated cells from the first suspension "continuously", this refers to a process in which the cells and preferably a fraction of the surrounding liquid is separated from the first suspension culture, while adding (fresh) liquid culture medium to the first suspension culture. Such process can for insance be performed when using a separation unit that is run in tangential flow filtration (TFF) mode. When continuously separating means separating the released differentiated cells from the first suspension "periodically/intermittently", this refers to a process in which the cells and preferably a fraction of the surrounding liquid is separated from the first suspension culture, followed by adding (fresh) liquid culture medium to the first suspension culture. Such process can for insance be performed when using a separation unit that is run in alternating tangential flow filtration (ATF) mode. Typically, in periodic/intermittent continuous separating the cells and preferably a fraction of the surrounding liquid is separated from the first suspension culture for a particular amount of time, followed by adding (fresh) liquid culture medium to the first suspension culture for a particular amount of time. Such amount of time may range from seconds to hours, preferably being in the range of 10 s to 100 min. Preferably, such amount of time is less than 24 hours, more preferably less than 12 hours, less than 6 hours or less than 3 hours.

According to one embodiment, continuously separating the released differentiated cells from the first suspension (such continuously or periodically/intermittently) results in a medium exchange rate of 0.01 to 2 vessel volumes per day, preferably 0.05 to 1 vessel volumes per day, more preferably 0.1 to 0.5 vessel volumes per day or 0.1 to 0.2 vessel volumes per day. Such medium exchange rates may depend on the scale of culture and in dependence of the differentiation and/or release rate of the differentiated cells. According to one embodiment, continuously separating the released differentiated cells from the first suspension (such continuously or periodically/intermittently) results in a medium exchange rate at least 0.01 vessel volumes per day, preferably 0.05 vessel volumes per day, more preferably 0.1 vessel volumes per day.

According to another embodiment, the porous-structured and/or micro-holed unit may comprise a coating suitable for retaining functional embryonic bodies. Any suitable coating that has previously been described and is known in the art for this purpose may be applied.

According to an embodiment, continuously separating the released differentiated cells may comprise obtaining the released differentiated cells constantly in a non-batch-wise manner without interrupting the culturing process. A non-batch-wise manner herein is different form a batch-wise manner, which typically refers to processes, wherein cells are cultured in a liquid medium being present in a container, e.g. bioreactor, and are not removed or are removed infrequently, e.g. only on a weekly or biweekly basis. In some embodiments, a non-batch-wise manner according to the present disclosure may include a periodic/intermittent separating the released differentiated cells, e.g. wherein cells and preferably a fraction of the surrounding liquid is separated from the first suspension culture for a particular amount of time, followed by adding (fresh) liquid culture medium to the first suspension culture for a particular amount of time. Such amount of time may range from seconds to hours, preferably being in the range of 10 s to 100 min. Preferably, such amount of time is less than 24 hours, more preferably less than 12 hours, less than 6 hours or less than 3 hours.

For example, it is contemplated that the method may be carried out in a closed system. "Closed system", as used herein refers to a culture vessel or bioreactor chamber and accessory components that have been pre-sterilized while closed and/or sealed and retains integrity and/or sterility. The vessels/bioreactor chambers and components are utilized without breach of the integrity of the system, permit fluid transfers in and/or out while maintaining asepsis, and are connectable to other closed systems without loss of integrity. A closed system bioreactor and/or vessel/bioreactor chamber refers to a system in which cells, cell culture medium, chemicals and reagents are aseptically added, removed and/or manipulated without breach of integrity of the system (e.g., by opening the cap of a tube or lifting the lid off a cell culture plate or dish). Single-use or multiple-use bags and/or containers and/or bioreactors in a closed system are added onto or into the closed system for example by sterile tube welding at the site of the vessel or bioreactor. Thus, performing the method in a closed system as herewith envisaged - among other advantageous - minimizes the potential for contamination, thereby improving safety of the process.

For example, the method may be performed in a tank bioreactor, such as a continuous stirred tank reactor, a rocking motion bioreactor, a wave bioreactor, an Erlenmeyer flask, a spinner flask, a rotating wall bioreactor, and/or a multi parallel bioreactor.

According to an embodiment, the separation step may comprise continuously transferring a liquid volume from the first suspension culture through the porous-structured and/or micro-holed unit.

Suspension culture upstream the porous-structured and/or micro-holed unit, in the context of this embodiment, may thus be referred to as first suspension culture, whereas suspension culture downstream the porous-structured and/or micro-holed unit may be referred to as second suspension culture. It is therefore contemplated that the first suspension culture in the context of the present invention may refer to the suspension culture comprising the at least one multicellular 3D aggregate. In such first suspension culture, single differentiated cells or smaller aggregates of differentiated cells that have been released from the at least one multicellular 3D aggregate into the first suspension culture may therefore e.g. still be in direct contact with the at least one multicellular 3D aggregate in the first suspension culture. As the term "second suspension culture" as used herein relates to the suspension culture downstream the porous-structured and/or micro-holed unit, single differentiated cells or smaller aggregates of differentiated cells in the second suspension unit can not be e.g. in direct contact with the at least one multicellular 3D aggregate in the first suspension culture. For example, a direct contact between single differentiated cells or smaller aggregates of differentiated cells in the second suspension unit with the at least one multicellular 3D aggregate may be impossible since the porous-structured and/or micro-holed unit may be construed to retain the at least one multicellular 3D aggregate in the first suspension culture (e.g. by selecting hole sizes smaller than the sizes of the at least one multicellular 3D aggregate in the first suspension culture).

According to an embodiment, the method may further comprise separating the differentiated cells from the at least one multicellular 3D aggregate by tangential flow filtration of the liquid volume in the separation unit.

According to an alternative embodiment, the method may further comprise separating the differentiated cells from the at least one multicellular 3D aggregate by alternating tangential flow filtration in the separation unit.

According to an alternative embodiment, the method may further comprise separating the differentiated cells from the at least one multicellular 3D aggregate by dead end filtration in the separation unit.

In a second aspect, the invention provides a cell population obtained by any one of the methods according to the present invention.

According to a third aspect, the present invention provides a pharmaceutical composition comprising the cells according to the present invention and a pharmaceutically acceptable carrier.

Pharmaceutical acceptable carriers are known in the art. It is herewith envisaged that the pharmaceutical composition may comprise any suitable carrier known in the art. For example, the pharmaceutical acceptable carrier may be a physiological saline solution. For example, if the differentiated cells are myeloid cells, the pharmaceutically acceptable carrier may typically be a buffer, e.g., PBS (PBS/EDTA supplemented with about 20% human serum albumin, or citrate plasma, or Plasmalyte-A pH 7.4 (Baxter, supplemented with about 2% human albumin). It is compatible with survival of the cells. It may comprise physiological levels of NaCl.

For example, the pharmaceutical composition may further comprise an adjuvant, excipient, buffer, diluent, carrier, stabilizer or combination thereof.

According to an embodiment, the cells and/or the pharmaceutical composition according to the present invention may be used for treating and/or preventing a disease.

According to another aspect of the invention, it is herewith provided a separation unit for separating differentiated cells from pluripotent cells, wherein the separation unit comprises a porous-structured and/or micro-holed unit, wherein the porous-structured and/or micro-holed unit is construed to being capable of separating a differentiated cell from a multicellular 3D aggregate.

According to another aspect of the invention, it is herewith provided a system for producing differentiated cells from pluripotent stem cells, comprising
- a bioreactor comprising at least one bioreactor chamber; the at least one bioreactor chamber having at least one fluid inlet and at least one fluid outlet;
- a separation unit in fluid communication with the at least one bioreactor chamber; the separation unit comprising at least one fluid inlet and one or more fluid outlets;

wherein a first fluid outlet of bioreactor chamber is continuously in fluid communication with a first inlet of the separation unit;
further wherein the separation unit comprises a porous-structured and/or micro-holed unit, wherein the porous-structured and/or micro-holed unit is construed to being capable of separating a differentiated cell from a multicellular 3D aggregate;
or wherein the separation unit comprises a sedimentation unit, wherein the sedimentation unit is construed to being capable of separating a differentiated cell from a multicellular 3D aggregate.

A bioreactor in accordance with the present invention may generally be understood to be an apparatus with a container (and/or bioreactor chamber) that can be used to carry out or support biotechnological processes, e.g. biopharmaceutical processes, from a laboratory to an industrial scale. In addition to bioreactors, in which mammalian cells may be cultivated under certain conditions, process mixing plants are encompassed.

The bioreactor system may be sterilizable, may maintain a sterile environment
The system may further include one or more of a mixing device, a gassing device, ports for sensors and for various operations like filling, harvesting, sampling and media addition/removal.

In some embodiments, a controller may monitor and/or control one or more parameters associated with the biotechnological process, like e.g. the critical process parameters (e.g. pH, DO and temperature). Mixing may e.g. be performed in stirred, rocking motion or orbital shaking mode.

According to an embodiment, the separation unit may be located inside or outside of the bioreactor chamber.

In some embodiment, the bioreactor chamber may be a dimensionally stable vessel made e.g. from stainless steel, glass or plastic material. Alternatively, it is envisaged that the bioreactor chamber may be a flexible bag made from at leat one suitable polymer, which may further be configured to be placed into a holder to safeguard dimensionally stability. In the art, the far most of the flexible bag containers as well as rigid plastic bioreactors are also known as single use bioreactors (SUB), which are encompassed herein.

According to an embodiment, the system may be suitable for carrying out a method according to the present invention. For example, at least one feature, preferably all features of the system may be construed to reduce (and/or avoid) any potential effect of mechanical cues on the differentiation of pluripotent stem cells.

According to an embodiment, the porous-structured and/or micro-holed unit may comprise a filter or a membrane.

In accordance with some embodiment, the porous-structured and/or micro-holed unit may comprise a hole diameter size of less than about 1000 µm, or less than about 500 µm, or less than about 200 µm, or less than about 150µm, or less than about 100 µm; and/or the porous-structured and/or micro-holed unit may comprise a hole diameter size of between about 100µm and 800 µm, between about 150 and 800 µm, between about 100 and 500 µm, between about 300 and 500 µm, between about 100 and 300 µm, between about 100 to 250 µm, between about 100 and 200 µm, or between about 100 and 150 µm.

According to an embodiment, the porous-structured and/or micro-holed unit may be produced by additive manufacturing such as Vat Photopolimerization (VP) or Powder Bed Fusion (PBF), e.g. Lithography-based Ceramics Manufacturing (LCM), Selective Laser Sintering (SLS), Stereolithography (SLS), or Digital Light Synthesis (DLS), by sintering, or by laser cutting.

It is herewith contemplated that the porous-structured and/or micro-holed unit may be made of a ceramic material, a stainless-steel material, a metal alloy material, an epoxy resin material, or a polymer material such as a thermoplastic material.

As described above with respect to the method of the present invention, advantageously, the use of additive manufacturing allows e.g. the provision of better defined cut-off values and/or smoother micro-hole edges, which are important parameters production of micro-holed units regarding suitability for retaining multicellular 3D aggregates in the first suspension culture without damaging their structure and/or function. For example, undesireable shear effects may occur if multicellular 3D aggregates interact with too small hole sizes of micro-holed units. Furthermore, this technology allows to change hole sizes according to the expected size of multicellular 3D aggregates which have to be retained, allows to select a specific orientation of holes in the micro-holed unit, and may introduce a porosity into the micro-hole unit. Without being bound to theory, it is believed that usage of a micro-holed unit produced by additive manufacturing methods may reduce the risk of any undesired activation of the multicellular 3D aggregates in the first suspension culture. For example, it has been described earlier that mechanical forces interfere with the development of embryonic tissues [Campas O., 2016]. It is thus important to control mechanical stimuli, including compression, tensile, or shear forces during culturing of the multicellular 3D aggregates in order to address the effect of mechanical cues on the differentiation of pluripotent stem cells.

According to another embodiment, the porous-structured and/or micro-holed unit may comprise a coating suitable for retaining functional embryonic bodies. Any suitable coating that has previously been described and is known in the art for this purpose may be applied.

According to an embodiment, the system may comprise at least one pump unit.

It is further envisaged that the at least one bioreactor chamber may further comprise at least one air outlet.

In some embodiments, a first outlet of the separation unit may be in fluid connection with a collector unit, the collector unit being constued to collect a permeate of the separation unit comprising the differentiated cells.

In accordance with some embodiments, the separation unit may comprise a tangential flow filtration unit.

In an embodiment, the system may be a closed system.

In some embodiments, the at least one bioreactor chamber may comprise a second fluid inlet, the second fluid inlet being in fluid communication with a second fluid outlet of the separation unit.

For example, the at least one pump unit may comprise a first circular pump unit, and further the first circular pump unit may be construed to continuously transfer liquid from the first fluid outlet of the bioreactor chamber to the fluid inlet of the separation unit, and further the first circular pump unit may be construed to continuously transfer the liquid from the first fluid inlet of the separation unit through the tangential flow filtration unit to the first and/or the second outlet of the separation unit.

In accordance with some embodiments, the at least one pump unit may comprise a first peristaltic pump unit, and the first peristaltic pump unit may be construed to continuously transfer the permeate of the separation unit comprising the differentiated cells to the collector unit.

In some embodiments, the system may comprise an alternating tangential flow (ATF) module. Each suitable ATF module known in the art may be implemented in the system of the present invention.

According to an embodiment, the separation unit may comprise a sedimentation unit.

For example, the sedimentation unit may comprise a container having at least one opening at the bottom.

The invention will be further illustrated by the following non-limiting Experimental Examples.

### Examples

An exemplary method according to a specific embodiment of the present invention is illustrated below:
Human induced pluripotent stem cells (iPSCs) are used for multicellular 3D aggregate creation (EB formation) and subsequent macrophage production. The exemplary process starts with a 3D priming of iPSCs in a well format, followed by a transfer of the EBs to a 2-liter stirred-tank bioreactor (STBR). Differentiation of cells into hematopoietic cells is subsequently induced. In the exemplary method, the differentiated macrophages are continuously obtained by using an alternating tangential flow unit (ATF):

### 3D Priming and Aggregate Selection:

The exemplary method may initiate with iPSCs undergoing 3D priming, leading to the formation of multicellular aggregates, referred to as EBs in the present example. These aggregates are incubated in a minimal iPSC medium, which typically contains essential nutrients and growth factors necessary for initial stages of cell differentiation. After the priming phase, the multicellular aggregates that exhibit optimal size and morphological characteristics, indicating readiness for further differentiation, are selected. The criteria for selecting suitable EBs are based on standardized assessments of aggregate diameter and cell density.

### Transfer to Bioreactor for Hematopoietic Specification:

The selected EBs are then carefully transferred to a 2-liter STBR. In this controlled environment, EBs are subjected to a differentiation medium, such as X-VIVO15, supplemented with key cytokines like IL-3 and M-CSF. The concentration of IL-3 is maintained within a range of 20-30 ng/ml, while M-CSF is kept at approximately 40-60 ng/ml. These concentrations are derived from standard practices in hematopoietic cell culture and are known to effectively induce macrophage differentiation. This differentiation medium may be referred to as an exemplary first suspension culture in accordance with the present invention.

### Bioreactor Condition Optimization:

Within the STBR, critical parameters such as pH (typically maintained between 7.2 and 7.4), dissolved oxygen (maintained at 40-50% of saturation), temperature (around 37°C), and agitation speed (optimized to prevent shear stress while ensuring uniform distribution of nutrients) are closely monitored and regulated. The bioreactor's internal environment is continuously monitored, with adjustments made as needed to ensure the EBs have the optimal conditions for growth and differentiation.

### Microholed ATF Unit for Macrophage Harvesting:

An ATF unit, comprising a micro-holed unit, is used in the exemplary method for the selective separation of macrophages from the larger EBs in the first suspension culture. The microholes in the micro-holed unit included in the ATF are precisely calibrated, typically ranging from 100 to 200 micrometers in diameter, to allow the passage of individual macrophages while retaining the EBs in the first suspension culture. The ATF unit's operation is fine-tuned to optimize the efficiency of macrophage separation, balancing the flow rate and shear forces to prevent damage to the cells.

### Continuous Harvesting/Separation Process:

As macrophages differentiate within the bioreactor, they are continuously harvested (obtained) through the micro-holed unit included in the ATF unit. Specifically, the differentiated macrophages are continuously separated from the EBs in the first suspension culture by continuously transferring the macrophages inside the ATF unit through the micro-holed unit. From another perspective, the differentiated macrophages are continuously transferred from the first suspension culture upstream the micro-holed unit to a second suspension culture downstream the micro-holed unit. Whereas in the first suspension culture, direct contact between differentiated macrophages and the EBs is possible, in the second suspension culture, the differentiated macrophages may no longer be in direct contact with the EBs (since the EBs cannot transfer the micro-holed unit because of their larger size). This step of continuous harvesting/separating the macrophages may not only involve the collection of macrophages but also their immediate assessment for quality and functionality, ensuring they meet predefined standards. The quality control of harvested macrophages includes assays for cell viability, purity, and specific macrophage markers to confirm their identity and functional capacity.

### Post-Harvest/Post-Separation Management and Quality Assurance:

During the continued method, the bioreactor and the ATF unit undergo a comprehensive maintenance protocol. This includes sterility checks and system integrity assessments to ensure readiness for ongoing cultivation. Quality control measures are rigorously applied throughout the process. This includes monitoring of bioreactor conditions, iPSC aggregate health, and the efficiency of the macrophage separation process

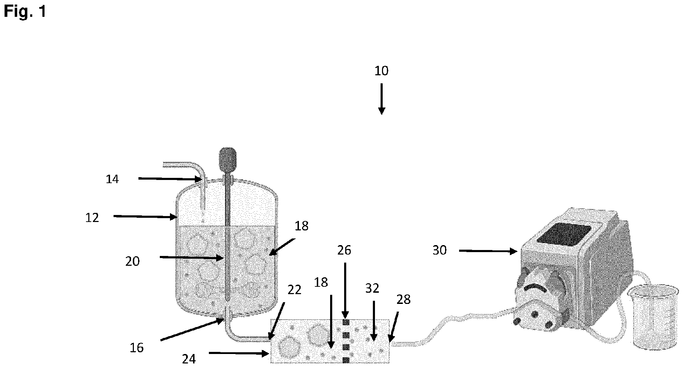

Fig. 1 shows an exemplary embodiment of a system 10 in accordance with the present invention. The system 10 represents a closed system and comprises a bioreactor including a bioreactor chamber 12. The bioreactor chamber 12 comprises a first fluid inlet 14 and a first fluid outlet 16. The first fluid inlet 14 may be in fluid communication with e.g. a further container, from which e.g. fresh culture medium may be transferred into the bioreactor chamber 12. In the bioreactor chamber 12 is a first suspension culture 18 comprising EBs, single differentiated cells and smaller aggregates of differentiated cells. The first suspension culture 18 may be continuously stirred by stirrer 20 of the system 10. The first fluid outlet 16 is in continuous fluid communication with a first fluid inlet 22 of a separation unit 24 of the system 10. The separation unit 24, in this embodiment as shown in Fig. 1, is located outside the bioreactor chamber 12. However, it is herewith contemplated that systems may be provided wherein the separation unit 24 may be located inside the bioreactor chamber 12.

The separation unit 24 comprises a micro-holed unit 26. The separation unit 24 further comprises a first fluid outlet 28. The first fluid outlet 28 of the separation unit 24 is in fluid communication with a collector unit. The system 10 further comprises a peristaltic pump 30. By means of the peristaltic pump 30 and possibly by means of optional further pumps which may not be shown in the Fig. 1, a continuous flow of liquid from the bioreactor chamber 12 into the separation unit 24, through the micro-holed unit 26 into the collector unit may be established. As described above, with respect to the present invention, the suspension culture upstream the micro-holed unit 26 is referred to as the first suspension culture 18, and a suspension culture downstream the micro-holed unit 26 is referred to as the second suspension culture 32. The hole sizes of the micro-holed unit 26 are selected to be suitable to retain EBs upstream of the micro-holed unit 26 in the first suspension culture 18. Furthermore, the hole sizes of the micro-holed unit 26 are selected to allow a transfer of single differentiated cells or of single differentiated cells and smaller aggregates of differentiated cells through the micro-holed unit 26 into the second suspension culture 32 (and thus into the collector unit). Thus, the exemplary system 10 of Fig. 1 enables a continuous separation of single differentiated cells and/or of single differentiated cells and smaller aggregates of differentiated cells from the first suspension culture, in which a direct contact between EBs and e.g. single differentiated cells is possible.

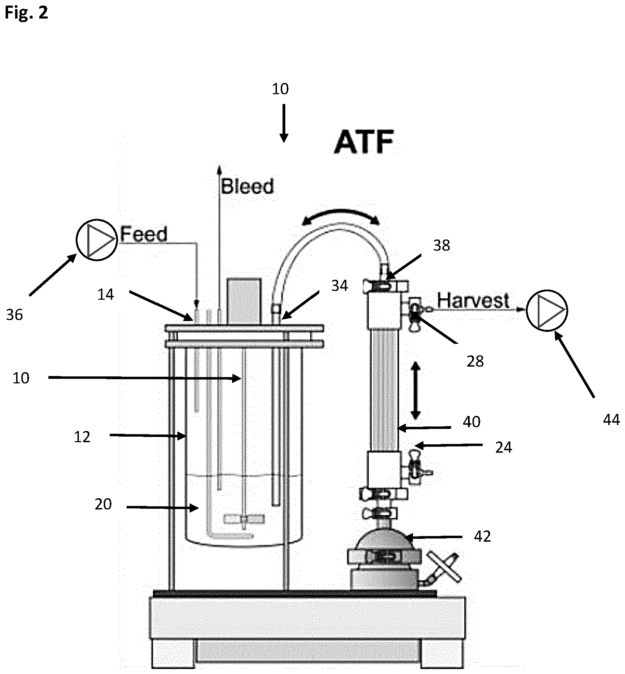

Fig. 2 shows another exemplary embodiment of a system 10 in accordance with the present invention. The system 10 represents a closed system and comprises a bioreactor including the bioreactor chamber 12. The bioreactor chamber 12 comprises the first fluid inlet 14 and a first fluid outlet 34. The first fluid inlet 14 may be in fluid communication with e.g. a further container, from which e.g. fresh culture medium may be transferred into the bioreactor chamber 12. Specifically, the system 10 as shown in Fig. 2 comprises a peristaltic pump 36 which is construed to transfer a feed into the bioreactor chamber 12. In the bioreactor chamber 12 is a first suspension culture 18 comprising EBs, single differentiated cells and smaller aggregates of differentiated cells. The first suspension culture 18 may be continuously stirred by stirrer 20 of the system 10. The first fluid outlet 34 is in continuous fluid communication with a first fluid inlet 38 of a separation unit 24 of the system 10. The separation unit 24, in this embodiment as shown in Fig. 2, is located outside the bioreactor chamber 12. However, it is herewith contemplated that systems may be provided wherein the separation unit 24 may be located inside the bioreactor chamber 12.

The separation unit 24 comprises an alternating tangential flow filtration module (ATM) 40. The ATM 40 includes the micro-holed unit 26. The ATM 40 further comprises a diaphragm pump 42 construed to alternate between positive pressure and vacuum to move a fluid back and forth across the micro-holed unit 26. The separation unit 24 further comprises the first fluid outlet 28. The first fluid outlet 28 of the separation unit 24 is in fluid communication with a collector unit. The system 10 further comprises a peristaltic pump 44. By means of the peristaltic pump 44, fluid which may have passed the micro-holed unit 26 in the ATM 40 may be transferred to the collector unit. By means of the diaphragm pump 42 and possibly by means of optional further pumps which may not be shown in the Fig. 2, a flow of liquid from the bioreactor chamber 12 into the separation unit 24, and - in the alternating mode - back into the bioreactor chamber 12 is established. In the alternating mode, the fluid inlet 38 of the separation unit 24 may serve as a fluid outlet, and the fluid outlet 34 of the chamber 12 may serve as a fluid inlet. Furthermore, during both flow directions established by the diaphragm pump 42 inside the ATM 40, a continuous tangential flow through the micro-holed unit 26 may be established. As described above, with respect to the present invention, the suspension culture upstream the micro-holed unit 26 is referred to as the first suspension culture 18, and a suspension culture downstream the micro-holed unit 26 is referred to as the second suspension culture 32. The hole sizes of the micro-holed unit 26 inside the ATM 40 are selected to be suitable to retain EBs upstream of the micro-holed unit 26 in the first suspension culture 18. Furthermore, the hole sizes of the micro-holed unit 26 are selected to allow a transfer of single differentiated cells or of single differentiated cells and smaller aggregates of differentiated cells through the micro-holed unit 26 into the second suspension culture 32 (and thus into the collector unit). Thus, the exemplary system 10 of Fig. 2 enables a continuous separation of single differentiated cells and/or of single differentiated cells and smaller aggregates of differentiated cells from the first suspension culture, in which a direct contact between EBs and e.g. single differentiated cells is possible, into the second suspension culture downstream the micro-holed unit 26, in which a direct contact between differentiated cells and Ebs is not possible.

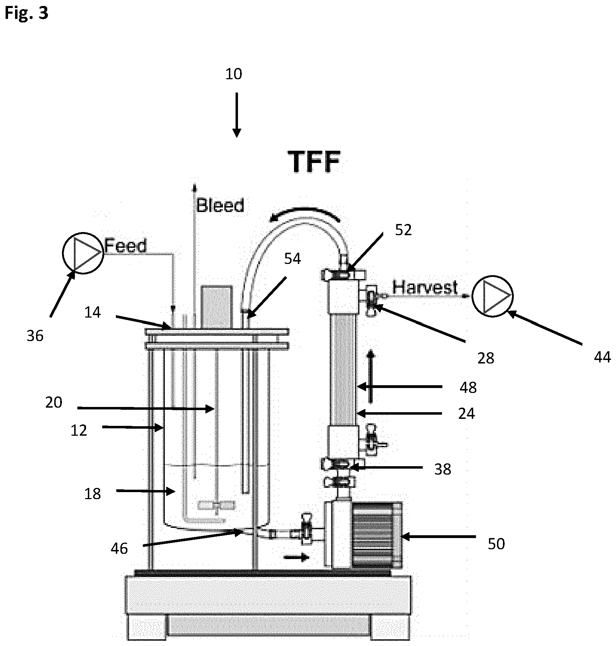

Fig. 3 shows another exemplary embodiment of a system 10 in accordance with the present invention. The system 10 represents a closed system and comprises a bioreactor including the bioreactor chamber 12. The bioreactor chamber 12 comprises the first fluid inlet 14 and a first fluid outlet 46. The first fluid inlet 14 may be in fluid communication with e.g. a further container, from which e.g. fresh culture medium may be transferred into the bioreactor chamber 12. Specifically, the system 10 as shown in Fig. 3 comprises a peristaltic pump 36 which is construed to transfer a feed into the bioreactor chamber 12. In the bioreactor chamber 12 is a first suspension culture 18 comprising EBs, single differentiated cells and smaller aggregates of differentiated cells. The first suspension culture 18 may be continuously stirred by stirrer 20 of the system 10. The first fluid outlet 46 is in continuous fluid communication with a first fluid inlet 38 of a separation unit 24 of the system 10. The separation unit 24, in this embodiment as shown in Fig. 3, is located outside the bioreactor chamber 12. However, it is herewith contemplated that systems may be provided wherein the separation unit 24 may be located inside the bioreactor chamber 12.

The separation unit 24 comprises a tangential flow filtration module (TFF) 48. The TFF 48 includes the micro-holed unit 26. The separation unit 24 further comprises the first fluid outlet 28. The first fluid outlet 28 of the separation unit 24 is in fluid communication with a collector unit. The separation unit 24 further comprises a second fluid outlet 52. The second fluid outlet 52 of the separation unit is in fluid communication with a second fluid inlet 54 of the bioreactor chamber 12. The TFF 48 further comprises a circular pump 50. By means of the circular pump 50, a continuous liquid flow may be established from the bioreactor chamber 12 into the TFF 48 and back into the bioreactor chamber 12. The system 10 further comprises a peristaltic pump 44. By means of the peristaltic pump 44, fluid which may have passed the micro-holed unit 26 in the TFF 48 may be transferred to the collector unit. Thus, during the flow of liquid established by the circular pump 50 inside the TFF 48, a continuous tangential flow through the micro-holed unit 26 may be established. As described above, with respect to the present invention, the suspension culture upstream the micro-holed unit 26 is referred to as the first suspension culture 18, and a suspension culture downstream the micro-holed unit 26 is referred to as the second suspension culture 32. The hole sizes of the micro-holed unit 26 inside the TFF 48 are selected to be suitable to retain EBs upstream of the micro-holed unit 26 in the first suspension culture 18. Furthermore, the hole sizes of the micro-holed unit 26 are selected to allow a transfer of single differentiated cells or of single differentiated cells and smaller aggregates of differentiated cells through the micro-holed unit 26 into the second suspension culture 32 (and thus into the collector unit). Thus, the exemplary system 10 of Fig. 3 enables a continuous separation of single differentiated cells and/or of single differentiated cells and smaller aggregates of differentiated cells from the first suspension culture, in which a direct contact between EBs and e.g. single differentiated cells is possible, into the second suspension culture downstream the micro-holed unit 26, in which a direct contact between differentiated cells and Ebs is not possible.

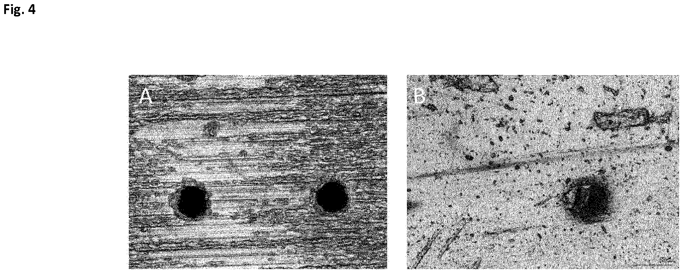

Fig. 4 shows scanning electron microscope image of an exemplary micro-holed unit of the system in accordance with some embodiments of the present invention; wherein Fig. 4A shows a micro-holed unit made of ceramic material with hole size of about 200 µm; and wherein Fig. 4B shows a micro-holed unit made of epoxy-resin material with hole size about 10 µm.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.
The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

In accordance with an embodiment, it is herewith provided a method of producing differentiated cells from pluripotent stem cells, comprising
- providing at least one multicellular 3D aggregate derived from pluripotent stem cells cultured in a first suspension culture;
- inducing differentiation of the cells forming the at least one multicellular 3D aggregate;
- allowing a continuous release of differentiated cells from the at least one multicellular 3D aggregate into the first suspension culture; and
- continuously separating the released differentiated cells from the first suspension culture;
wherein continuously separating the released differentiated cells comprises separating the released differentiated cells constantly in a non-batch-wise manner without interrupting the culturing process; and further wherein the method is carried out in a closed system.

According to a further embodiment, there is provided a method of producing differentiated cells from pluripotent stem cells, comprising
- providing at least one multicellular 3D aggregate derived from pluripotent stem cells cultured in a first suspension culture;
- inducing differentiation of the cells forming the at least one multicellular 3D aggregate;
- allowing a continuous release of differentiated cells from the at least one multicellular 3D aggregate into the first suspension culture; and
- continuously separating the released differentiated cells from the first suspension culture;
wherein continuously separating the released differentiated cells comprises separating the released differentiated cells constantly in a non-batch-wise manner without interrupting the culturing process; optionally wherein the method is carried out in a closed system; wherein the step of separating the differentiated cells from the at least one embryoid body comprises passing the first suspension culture through a separation unit, the separation unit being construed to separate components of the first suspension culture according to size; wherein the separation unit comprises a porous-structured and/or micro-holed unit, such as a filter or membrane; optionally wherein the porous-structured and/or micro-holed unit comprises a hole diameter size of between about 100 and 300 µm, preferably between about 100 to 250 µm, more preferably between about 100 and 200 µm.

According to a further embodiment, there is provided a method of producing differentiated cells from pluripotent stem cells, comprising
- providing at least one multicellular 3D aggregate derived from pluripotent stem cells cultured in a first suspension culture;
- inducing differentiation of the cells forming the at least one multicellular 3D aggregate;
- allowing a continuous release of differentiated cells from the at least one multicellular 3D aggregate into the first suspension culture; and
- continuously separating the released differentiated cells from the first suspension culture;
wherein continuously separating the released differentiated cells comprises separating the released differentiated cells constantly in a non-batch-wise manner without interrupting the culturing process; optionally wherein the method is carried out in a closed system; wherein the step of separating the differentiated cells from the at least one embryoid body comprises passing the first suspension culture through a separation unit, the separation unit being construed to separate components of the first suspension culture according to size; wherein the separation unit comprises a porous-structured and/or micro-holed unit, such as a filter or membrane; optionally wherein the porous-structured and/or micro-holed unit comprises a hole diameter size of between about 100 and 300 µm, preferably between about 100 to 250 µm, more preferably between about 100 and 200 µm; and further wherein the porous-structured and/or micro-holed unit is produced by additive manufacturing, preferably wherein the porous-structured and/or micro-holed unit is monolithic.

According to a further embodiment, there is provided a method of producing differentiated cells from pluripotent stem cells, comprising
- providing at least one multicellular 3D aggregate derived from pluripotent stem cells cultured in a first suspension culture;
- inducing differentiation of the cells forming the at least one multicellular 3D aggregate;
- allowing a continuous release of differentiated cells from the at least one multicellular 3D aggregate into the first suspension culture; and
- continuously separating the released differentiated cells from the first suspension culture;
wherein continuously separating the released differentiated cells comprises separating the released differentiated cells constantly in a non-batch-wise manner without interrupting the culturing process; optionally wherein the method is carried out in a closed system; wherein the step of separating the differentiated cells from the at least one embryoid body comprises passing the first suspension culture through a separation unit, the separation unit being construed to separate components of the first suspension culture according to size; wherein the separation unit comprises a porous-structured and/or micro-holed unit, such as a filter or membrane; optionally wherein the porous-structured and/or micro-holed unit comprises a hole diameter size of between about 100 and 300 µm, preferably between about 100 to 250 µm, more preferably between about 100 and 200 µm; and further wherein the porous-structured and/or micro-holed unit is produced by additive manufacturing, preferably wherein the porous-structured and/or micro-holed unit is monolithic; further comprising separating the differentiated cells from the embryonic bodies by alternating tangential flow filtration in the separation unit.

The invention is further characterized by the following items:
1. A method of producing differentiated cells from pluripotent stem cells, comprising
   - providing at least one multicellular 3D aggregate derived from pluripotent stem cells cultured in a first suspension culture;
   - inducing differentiation of the cells forming the at least one multicellular 3D aggregate;
   - allowing a continuous release of differentiated cells from the at least one multicellular 3D aggregate into the first suspension culture;
   - continuously separating the released differentiated cells from the first suspension culture.
2. The method according to item 1, wherein continuously obtaining the released differentiated cells comprises obtaining the released differentiated cells constantly in a non-batch-wise manner without interrupting the culturing process.
3. The method according to any one of the proceeding items, wherein the method is carried out in a closed system.
4. The method according to anyone of the foregoing items, wherein the released differentiated cells comprise single differentiated cells, or cell aggregates of the differentiated cells.
5. The method according to any one of the proceeding items, wherein the at least one multicellular 3D aggregate comprises the pluripotent stem cells, or progenitor cells derived from the pluripotent stem cells, or a mixture thereof.
6. The method according to any one of the proceeding items, wherein the stem cells are induced pluripotent stem cells (iPSC) or embryonic stem cells.
7. The method according to any one of the proceeding items, wherein the stem cells are human stem cells.
8. The method according to any one of the proceeding items, wherein the produced differentiated cells are partially or terminally differentiated cells.
9. The method according to any one of the proceeding items, wherein the produced differentiated cells are myeloid cells.
10. The method according to any one of the proceeding items, wherein continuously separating the differentiated cells from the first suspension culture comprises the step of separating the differentiated cells from the at least one multicellular 3D aggregate.
11. The method according to any one of the proceeding items, wherein wherein continuously separating the differentiated cells from the first suspension culture comprises continuously or periodically/intermittently exchanging a culture medium of the first suspension culture; wherein optionally a rate of the medium exchange is between about 0.01 to about 2 vessel volumes per day, preferably between about 0.05 to about 1 vessel volumes per day, more preferably between about 0.1 to about 0.5 vessel volumes per day or between about 0.1 to about 0.2 vessel volumes per day.
12. The method according to item 10 ot item 11, wherein the step of separating the differentiated cells from the at least one multicellular 3D aggregate comprises passing the first suspension culture through a separation unit, the separation unit being construed to separate components of the first suspension culture according to size.
13. The method according to item 10 or item 11, wherein the step of separating the differentiated cells from the at least one multicellular 3D aggregate comprises separating components of the first suspension culture according to density.
14. The method according to item 12, wherein the separation unit comprises a porous-structured and/or micro-holed unit, such as a filter or membrane.
15. The method according to item 14, wherein the porous-structured and/or micro-holed unit comprises a hole diameter size of less than about 1000 µm, or less than about 500 µm, or less than about 200 µm, or less than about 150µm, or less than about 100 µm; and/or wherein the porous-structured and/or micro-holed unit comprises a hole diameter size of between about 100µm and 800 µm, between about 150 and 800 µm, between about 100 and 500 µm, between about 300 and 500 µm, between about 100 and 300 µm, between about 100 to 250 µm, between about 100 and 200 µm, or between about 100 and 150 µm.
16. The method according to any one of items 14-15, wherein the porous-structured and/or micro-holed unit is produced by additive manufacturing such as Vat Photopolimerization (VP) or Powder Bed Fusion (PBF), e.g. Lithography-based Ceramics Manufacturing (LCM), Selective Laser Sintering (SLS), Stereolithography (SLS), or Digital Light Synthesis (DLS), by sintering, or by laser cutting.
17. The method according to any one of items 12 or 14-16, wherein the porous-structured and/or micro-holed unit is made of a ceramic material, a stainless steel material, a metal alloy material, an epoxy resin material, or a polymer material such as a thermoplastic material.
18. The method according to any one of items 12 or 14-17, wherein the porous-structured and/or micro-holed unit comprises a coating suitable for retaining functional multicellular 3D aggregates.
19. The method according to any one of items 12 or 14-18, wherein the porous-structured and/or micro-holed unit is monolithic.
20. The method according to any one of the proceeding items, wherein the method is carried out in a tank bioreactor, such as a continuous stirred tank reactor, a rocking motion bioreactor, a wave bioreactor, an Erlenmeyer flask, a spinner flask, a rotating wall bioreactor, and/or a multi parallel bioreactor.
21. The method according to any one of items 12 or 14-20, wherein the separation step comprises continuously transferring a liquid volume from the first suspension culture through the porous-structured and/or micro-holed unit.
22. The method according to item 21, comprising separating the differentiated cells from the embryonic bodies by tangential flow filtration of the liquid in the separation unit.
23. The method according to item 21, comprising separating the differentiated cells from the embryonic bodies by alternating tangential flow filtration in the separation unit.
24. The method according to item 21, comprising separating the differentiated cells from the embryonic bodies by dead end filtration in the separation unit.
25. A cell population obtained by any one of the methods according to items 1-24.
26. A pharmaceutical composition comprising the cells according to item 25 and a pharmaceutically acceptable carrier.
27. Use of the cells according to item 25 or the composition according to item 26 for treating or preventing a disease.
28. A separation unit for separating differentiated cells from pluripotent cells, wherein the separation unit comprises a porous-structured and/or micro-holed unit, wherein the porous-structured and/or micro-holed unit is construed to being capable of separating a differentiated cell from a multicellular 3D aggregate.
29. A system for producing differentiated cells from pluripotent stem cells, comprising
   - a bioreactor comprising at least one bioreactor chamber; the at least one bioreactor chamber having at least one fluid inlet and at least one fluid outlet;
   - a separation unit in fluid communication with the at least one bioreactor chamber; the separation unit comprising at least one fluid inlet and one or more fluid outlets;

   wherein a first fluid outlet of bioreactor chamber is continuously in fluid communication with a first inlet of the separation unit;
   further wherein the separation unit comprises a porous-structured and/or micro-holed unit, wherein the porous-structured and/or micro-holed unit is construed to being capable of separating a differentiated cell from a multicellular 3D aggregate;
   or wherein the separation unit comprises a sedimentation unit, wherein the sedimentation unit is construed to being capable of separating a differentiated cell from a multicellular 3D aggregate.
30. The system according to item 29, wherein the separation unit is located inside the bioreactor chamber.
31. The system according to item 29, wherein the separation unit is located outside of the bioreactor chamber.
32. The system according to any one of items 29-31, wherein the system is suitable for carrying out a method according to any one of the items 1-24.
33. The system according to any one of items 29-32 or the separation unit according to item 28, wherein the porous-structured and/or micro-holed unit comprises a filter or a membrane.
34. The system according to any one of items 29-33 or the separation unit according to item 28, wherein the porous-structured and/or micro-holed unit comprises a hole diameter size of less than about 1000 µm, or less than about 500 µm, or less than about 200 µm, or less than about 150µm, or less than about 100 µm; and/or wherein the porous-structured and/or micro-holed unit comprises a hole diameter size of between about 100µm and 800 µm, between about 150 and 800 µm, between about 100 and 500 µm, between about 300 and 500 µm, between about 100 and 300 µm, between about 100 to 250 µm, between about 100 and 200 µm, or between about 100 and 150 µm.
35. The system according to any one of items 29-34 or the separation unit according to item 28, wherein the porous-structured and/or micro-holed unit is produced by additive manufacturing such as Vat Photopolimerization (VP) or Powder Bed Fusion (PBF), e.g. Lithography-based Ceramics Manufacturing (LCM), Selective Laser Sintering (SLS), Stereolithography (SLS), or Digital Light Synthesis (DLS), by sintering, or by laser cutting.
36. The system according to any one of items 29-35 or the separation unit according to item 28, wherein the porous-structured and/or micro-holed unit is made of a ceramic material, a stainless steel material, a metal alloy material, an epoxy resin material, or a polymer material such as a thermoplastic material.
37. The system according to any one of items 29-36 or the separation unit according to item 28, wherein the porous-structured and/or micro-holed unit is monolithic.
38. The system according to any one of items 29-37 or the separation unit according to item 28, wherein the separation unit comprises a coating suitable for retaining functional embryonic bodies.
39. The system according to any one of items 29-38, further comprising at least one pump unit.
40. The system according to any one of items 29-39, wherein the at least one bioreactor chamber further comprises at least one air outlet.
41. The system according to any one of items 29-40, wherein a first outlet of the separation unit being in fluid connection with a collector unit, the collector unit being construed to collect a permeate of the separation unit comprising the differentiated cells.
42. The system according to any one of items 29-40, wherein the separation unit comprises a tangential flow filtration unit.
43. The system according to any one of the items 29-42; wherein the system is a closed system.
44. The system according to any one of items 29- 43, wherein the at least one bioreactor chamber comprises a second fluid inlet, the second fluid inlet being in fluid communication with a second fluid outlet of the separation unit.
45. The system according to item 44, wherein the at least one pump unit comprises a first circular pump unit, and further wherein the first circular pump unit is construed to continuously transfer liquid from the first fluid outlet of the bioreactor chamber to the fluid inlet of the separation unit, further wherein the first circular pump unit is construed to continuously transfer the liquid from the first fluid inlet of the separation unit through the tangential flow filtration unit to the first and/or the second outlet of the separation unit.
46. The system according to any one of items 39-44, wherein the at least one pump unit comprises a first peristaltic pump unit, and further wherein the first peristaltic pump unit is construed to continuously transfer the permeate of the separation unit comprising the differentiated cells to the collector unit.
47. The system according to any one of items 39-44 or 46, further comprising an alternating tangential flow (ATF) module.
48. The system according to any one of items 26-43, wherein the separation unit comprises a sedimentation unit.
49. The system according to item 48, wherein the sedimentation unit comprises a container having at least one opening at the bottom.

## Claims

1. A method of producing differentiated cells from pluripotent stem cells, comprising
- providing at least one multicellular three-dimensional (3D) aggregate derived from pluripotent stem cells cultured in a first suspension culture;
- inducing differentiation of the cells forming the at least one multicellular 3D aggregate;
- allowing a continuous release of differentiated cells from the at least one multicellular 3D aggregate into the first suspension culture; and
- continuously separating the released differentiated cells from the first suspension culture.

2. The method according to claim 1, wherein continuously separating the released differentiated cells comprises separating the released differentiated cells constantly in a non-batch-wise manner without interrupting the culturing process.

3. The method according to any one of the proceeding claims, wherein the method is carried out in a closed system; optionally wherein the method is carried out in a tank bioreactor, such as a continuous stirred tank reactor, a rocking motion bioreactor, a wave bioreactor, an Erlenmeyer flask, a spinner flask, a rotating wall bioreactor, and/or a multi parallel bioreactor.

4. The method according to any one of the proceeding claims, wherein the released differentiated cells comprise:
- single differentiated cells, and/or
- cell aggregates of the differentiated cells;
and/or
wherein the multicellular 3D aggregate comprises:
- the pluripotent stem cells, or
- progenitor cells derived from the pluripotent stem cells, or
- a mixture thereof;
and/or
wherein the stem cells are:
- induced pluripotent stem cells (iPSC);
- embryonic stem cells; and/or
- human stem cells;
and/or
wherein the produced differentiated cells are:
partially or terminally differentiated cells, such as differentiated myeloid cells.

5. The method according to any one of the proceeding claims, wherein continuously separating the differentiated cells from the first suspension culture comprises:
- continuously or periodically/intermittently exchanging a culture medium of the first suspension culture, wherein optionally a rate of the medium exchange is between about 0.01 to about 2 vessel volumes per day, preferably between about 0.05 to about 1 vessel volumes per day, more preferably between about 0.1 to about 0.5 vessel volumes per day or between about 0.1 to about 0.2 vessel volumes per day;
and/or
- continuously separating the differentiated cells from the at least one multicellular 3D aggregate; optionally wherein the step of separating the differentiated cells from the at least one multicellular 3D aggregate comprises:
- passing the first suspension culture through a separation unit, the separation unit being construed to separate components of the first suspension culture according to size; or
- separating components of the first suspension culture according to density.

6. The method according to claim 5, wherein the separation unit comprises a porous-structured and/or micro-holed unit, such as a filter or membrane; optionally wherein the porous-structured and/or micro-holed unit has one or more of the following features:
- it is monolithic;
- it comprises a hole diameter size of less than about 1000 µm, or less than about 500 µm, or less than about 200 µm, or less than about 150µm, or less than about 100 µm; and/or wherein the porous-structured and/or micro-holed unit comprises a hole diameter size of between about 100µm and 800 µm, between about 150 and 800 µm, between about 100 and 500 µm, between about 300 and 500 µm, between about 100 and 300 µm, between about 100 to 250 µm, between about 100 and 200 µm, or between about 100 and 150 µm;
- it is produced by additive manufacturing such as Vat Photopolimerization (VP) or Powder Bed Fusion (PBF), e.g. Lithography-based Ceramics Manufacturing (LCM), Selective Laser Sintering (SLS), Stereolithography (SLS), or Digital Light Synthesis (DLS), by sintering, or by laser cutting;
and/or
- it is made of a ceramic material, a stainless-steel material, a metal alloy material, an epoxy resin material, or a polymer material such as a thermoplastic material; and/or
- it comprises a coating suitable for retaining functional multicellular 3D aggregates.

7. The method according to any one of claims 5 to 6, wherein the separation step comprises continuously transferring a liquid volume from the first suspension culture through the porous-structured and/or micro-holed unit; optionally wherein the method further comprises separating the differentiated cells from the at least one multicellular 3D aggregate by
- tangential flow filtration of the liquid in the separation unit;
- alternating tangential flow filtration in the separation unit; or
- by dead end fitration in the separation unit.

8. A cell population obtained by any one of the methods according to claims 1-7.

9. A pharmaceutical composition comprising the cells according to claim 8 and a pharmaceutically acceptable carrier.

10. The cells according to claim 8 or the composition according to claim 9 for use in treating or preventing a disease.

11. A separation unit for separating differentiated cells from pluripotent cells, wherein the separation unit comprises a porous-structured and/or micro-holed unit, wherein the porous-structured and/or micro-holed unit is construed to being capable of separating a differentiated cell from a multicellular 3D aggregate.

12. A system for producing differentiated cells from pluripotent stem cells, comprising
- a bioreactor comprising at least one bioreactor chamber; the at least one bioreactor chamber having at least one fluid inlet and at least one fluid outlet;
- a separation unit in fluid communication with the at least one bioreactor chamber; the separation unit comprising at least one fluid inlet and one or more fluid outlets;
wherein a first fluid outlet of bioreactor chamber is continuously in fluid communication with a first inlet of the separation unit;
further wherein the separation unit comprises a porous-structured and/or micro-holed unit, wherein the porous-structured and/or micro-holed unit is construed to being capable of separating a differentiated cell from a multicellular 3D aggregate;
or wherein the separation unit comprises a sedimentation unit, wherein the sedimentation unit is construed to being capable of separating a differentiated cell from a multicellular 3D aggregate; optionally wherein the sedimentation unit comprises a container having at least one opening at the bottom.

13. The system according to claim 12, wherein the system is suitable for carrying out a method according to any one of the claims 1-7.

14. The system according to any one of claims 12 or 13, or the separation unit according to claim 11, having one or more of the following features:
- wherein the porous-structured and/or micro-holed unit is monolithic;
- wherein the porous-structured and/or micro-holed unit comprises a filter or a membrane;
- wherein the porous-structured and/or micro-holed unit comprises a hole diameter size of less than about 1000 µm, or less than about 500 µm, or less than about 200 µm, or less than about 150µm, or less than about 100 µm;
- wherein the porous-structured and/or micro-holed unit comprises a hole diameter size of between about 100µm and 800 µm, between about 150 and 800 µm, between about 100 and 500 µm, between about 300 and 500 µm, between about 100 and 300 µm, between about 100 to 250 µm, between about 100 and 200 µm, or between about 100 and 150 µm;
- wherein the porous-structured and/or micro-holed unit is produced by additive manufacturing such as Vat Photopolimerization (VP) or Powder Bed Fusion (PBF), e.g. Lithography-based Ceramics Manufacturing (LCM), Selective Laser Sintering (SLS), Stereolithography (SLS), or Digital Light Synthesis (DLS), by sintering or by laser cutting;
- wherein the the porous-structured and/or micro-holed unit is made of a ceramic material, a stainless-steel material, a metal alloy material, an epoxy resin material, or a polymer material such as a thermoplastic material; and/or
- wherein the separation unit comprises a coating suitable for retaining functional embryonic bodies.

15. The system according to any one of claims 12-14, further comprising one or more of the following:
- at least one pump unit;
- wherein the at least one bioreactor chamber further comprises at least one air outlet;
- wherein a first outlet of the separation unit being in fluid connection with a collector unit, the collector unit being construed to collect a permeate of the separation unit comprising the differentiated cells;
- wherein the system is a closed system; and/or
- wherein the separation unit comprises a tangential flow filtration (TFF) module or an alternating tangential flow (ATF) module.
